# EUROPEAN PATENT SPECIFICATION

(11) **EP 1 886 632 B1**
(45) Date of publication and mention of the grant of the patent: **06.04.2011**
(21) Application number: 07019614.2
(22) Date of filing: 10.08.2000
(51) Int. Cl.: A61B 17/12, A61F 2/06

(54) **Intravascular delivery system**
System zur intravascularen Implantation
Système de mise en place intra-vasculaire

(43) Date of publication of application: 13.02.2008
(62) Divisional of application: 00953957.8
(73) Proprietor: Micrus Endovascular Corporation, San Jose, CA 95131 (US)
(72) Inventor: Kurz, Daniel R., Monterey, CA 93940 (US)
(74) Representative: McLeish, Nicholas Alistair Maxwell

(56) References cited:
- EP-A- 0 717 969
- WO-A-00/12015
- WO-A-93/11823
- WO-A-99/32037
- US-A- 5 609 608

## Description

### BACKGROUND OF THE INVENTION

### Field of the Invention:

This invention relates generally to therapeutic placement of interventional medical devices into the vasculature of the human body. More particularly, this invention concerns a placement system using a catheter with a tip formed of yieldable material to grip and thereby releasably capture a portion of an endoluminal device to be dislodged at a desired location within the body by a device for dislodging the endoluminal device from the catheter tip.

### Description of Related Art:

A type of interventional medical device known as an endoluminal coil is used for a wide variety of therapeutic purposes including the treatment of intracranial vascular aneurysms. A vascular aneurysm is often formed as the result of an abnormal dilation of a blood vessel which weakens the arterial wall and allows it to expand into an adjacent body tissue or cavity. Intracranial aneurysms may be treated to prevent rupturing by placing endoluminal coils through the neck of an opening from the vessel into the interior cavity of the aneurysm. After placement, the coils pose a physical barrier, reducing blood flow into the aneurysm and promoting the formation of an embolus in the aneurysm cavity. The embolus formation in the aneurysm cavity further impedes blood flow into the aneurysm and reduces the blood pressure against the aneurysm wall, thus reducing the possibility of a rupture.

One known method for delivering coils into an intracranial aneurysm involves the use of a catheter and a guidewire with a detachable tip shaped in the form of a coil.

Microcatheters are known, for example, that allow for navigation into cerebral arteries and entry into intracranial aneurysms. The catheter is guided through the vasculature using a guidewire until it reaches the desired location. The tip of the guidewire is then detached and the coils are pushed into the aneurysm until they occlude at least a portion of the interior of the aneurysm. Although generally effective, this technique has limitations on the accuracy for precision placement of embolic coils in intracranial aneurysms. It would be particularly desirable to have a simple delivery system which allows for precise positioning of embolic coils and virtually instantaneous release once the coils are in place.

One approach which provides for greater accuracy of placement involves attaching a coil to the end of the a guidewire and maneuvering the guidewire to place the embolic coil in the desired location and then releasing the coil from the guidewire. Since the physician has control of the guidewire and the coil is firmly attached to the distal end of the pusher, it is possible to achieve a much higher degree of placement accuracy with this approach. However, to implement this approach, the delivery system must include a release mechanism which can be used to easily decouple the coil from the guidewire while inside tiny blood vessels. A variety of release mechanisms have been proposed for this purpose.

In one known technique for deploying an endoluminal device, endoluminal coils may be released through electrolytic dissolution of a connecting segment between the coil and the distal tip of the guidewire. This method typically involves the application of a positive direct current for a predetermined amount of time which results in the dissolution of a stainless steel connector which holds the coil to the guidewire. Although this method has met with considerable success, the procedure has significant disadvantages. Because the use of electrolytic dissolution is slow and unreliable, the delivery of the devices can be very time consuming and therefore very costly. The increased surgery time also creates a higher risk for the patient. In addition, the secondary effects of dissolving a stainless steel wire in the blood could possibly be detrimental to the patient. For these reasons, a simpler, faster, safer and more reliable method of delivering the devices is needed.

Detachable coil assemblies are also known that use a threaded coupling such that the coil is released when the guidewire is rotated. Another conventional technique uses a heat-releasable adhesive bond to separate the coils from the distal end of the catheter. When laser energy is transferred via a fiber optic cable to the connector, the connector is heated, thereby releasing the heat-sensitive adhesive bond between the connector and coil.

One known implant delivery assembly is activated thermally, and includes a coupling portion made of a shape memory material that interlockingly engages the implant when the shape memory material is in one configuration, and releases the implant in another configuration. The implant is detachably coupled to a pusher formed of shape memory material that allows thermal activation of the decoupling mechanism. The coupling portion is constructed with a deformed shape for holding the implant to the pusher, and a pre-set shape that provides release of the implant when the thermal activation is provided. The coupling portion of the pusher is heated by passing an electric current between the pusher and the body of the patient.

Another detachable embolic coil assembly is known that uses interlocking clasps that are used in a surgical instrument for delivering an embolic coil to a selected site within the vasculature of the human body.

Yet another known embolic coil assembly includes a ball that is forced through an aperture in a socket on the distal end of a pusher to release the coil. After a catheter is inserted and navigated through the vessel, and the coil is in place, a plunger is advanced to press the ball and its coil into the target site.

Some conventional vasoocclusive devices are operated by pulling or jerking the catheter tip from an inflatable balloon, thus potentially compromising the position of the implant. One such device provides for an endovascular wire and tip that can be separated from the holding wire mechanically or electrolytically for the formation of thrombus in blood vessels. However, such devices that release the interventional device by mechanically breaking an intermediate section between the catheter tip and balloon can potentially leave broken or jagged ends that could injure the vasculature.

One conventional releasable balloon catheter used to embolize vascular lesions has a tubular portion made of a material such as a hydrophilic polymer located between the catheter and the balloon that can be broken by torsion of the tubular portion. The tubular portion can be melted by heating the tubular portion, or can be dissolved in the blood when heated, and electrodes are provided for heating the tubular portion. Another conventional technique for separating a balloon from a balloon catheter involves the melting and breaking of a connecting member made from polyvinyl alcohol or trans-polyisoprene between the balloon and the catheter body when power is supplied to electrodes provided for heating the connecting member. When the connecting member is heated to temperatures of about70°C and slight tension is applied, the balloon can be separated from the main catheter body. However, such devices that release the interventional device by melting or dissolving the intermediate section between the catheter tip and balloon can also potentially release undesirable particles from the connecting member into the bloodstream.

From the above, it can be seen that a variety of approaches to placing embolic devices have been developed, but all of them are limited in some way by the time to release, the dispersion of particles or chemicals, the introduction of electricity, mechanical force on the implant after placement, or some combination of these affects. There is therefore a need for a precise, controlled method of deploying therapeutic interventional devices without compromising the position of the implant, without presenting broken or jagged ends that can potentially injure the vasculature, and without release undesirable particles or materials into the bloodstream.

Recently, a release system for vasoocclusive coils has been developed involving the use of a microgripper made of shape memory material. The shape memory microgrippers is mechanically actuated by the conversion of laser light to heat energy. Another newly developed type of release mechanism using shape memory materials involves a tube of radially recovering shape memory polymer attached to the distal end of an optical fiberpusher. A device such as an endoluminal coil is introduced into the tube and the tube is compressed or crimped around the end of the coil to hold it in place. Once the coil is in the desired location in the vasculature, the tube of shape memory polymer is heated by passing light through the optical fiber pusher to the distal end of the pusher, thereby causing the tube to recover its original diameter and shape. After the tube has recovered its original shape, it is no longer compressed or crimped around the device and the device is free to slip out of the tube.

In another approach, an endoluminal coil delivery system is provided with a mechanical release mechanism for positioning and delivering a coil within a lumen that utilizes a mechanical latch to engage the coil during positioning. The coil is placed at the distal end of delivery system and includes a fitting at the end of coil which is engaged by jaws. The coil is released from the jaws by advancing a release tube over the jaws, which squeezes the jaws, thereby disengaging them from the fitting.

Another endoluminal coil delivery system utilizes an elongated pusher member with a coil implant detachably coupled to a relatively short flexible distal section of the pusher member by a curved coupling portion of the pusher member that can be thermally activated to transform to a preset release configuration. In another coil delivery system, embolic coils are threaded onto a guide wire ahead of a pusher located within a catheter, allowing several coils to be loaded on the guide wire. In another endoluminal coil delivery system, an end of a vasoocclusive coil is open and adapted to receive and engage a conical or cylindrical tip of a pusher core wire. The tip of the pusher wire may be smooth, grooved or in a semi-machined or sanded condition to achieve good adherence of the pusher core wire to the interior of the vasoocclusive coil.

WO99/32037 discloses a coil-delivery device for delivery of a vaso-occlusion coil to a vascular target site via a catheter. The device includes a wire adapted to be slideably received within the lumen of a catheter or an introducer, the wire having at its distal end a stiff wavy wire segment adapted to fractionally and releaseably engage a vaso-occlusion coil by the end-region inner lumen of the vaso-occlusion coil. The coil-delivery device may include a radial enlargement which separates the wavy wire segment from upstream portions of the device.

A shape memory metal actuated separation device is also known that can be used for spacecraft. A segmented nut engages a threaded bolt that is to be held and released and is held together by a nut retainer that is movable with respect to the nut and affixed to a shape memory alloy element. The shape memory alloy element is heated by an electrical resistance heater, thereby moving the retainer which causes disengagement.

In one coil shaped intravascular stent formed into a coil spring, to be used to reinforce an arterial wall, the wire forming the stent has axially spaced rollers or bearings to facilitate advancement and withdrawal of the coil spring, with enlarged beads between the rollers to hold the rollers away from one another.

Thus, it can be seen that there is a continued requirement for reliable vascular device and embolic coil release systems. The present invention meets these and other needs.

### SUMMARY OF THE INVENTION

According to the present invention there is disclosed an endoluminal device delivery assembly as claimed in the appended claims.

Briefly, and in general terms, the present invention provides for an improved system for release and deployment of an endoluminal therapeutic device at a desired location for treatment within the vasculature of a patient, utilizing a yieldable material for releasably holding a portion of the endoluminal therapeutic device, to be dislodged from the yieldable material at the desired location for treatment within the vasculature of a patient.

The invention accordingly provides for an endoluminal device delivery assembly for release and deployment of an endoluminal therapeutic device at a desired location for treatment within the vasculature of a patient, comprising an elongated flexible tubular catheter having a distal end, and a tubular distal tip having a proximal end mounted to the distal end of the catheter, an inner lumen, and a distal end with a surface defining a distal opening, and the tubular distal tip being formed of a yieldable material for releasably holding the proximal end of the endoluminal device within the inner lumen of the tubular distal tip. Means are also provided for dislodging the proximal end of the endoluminal device from the inner lumen of the tubular distal tip to expel the proximal end of the endoluminal device through the distal opening of the tubular distal tip at the desired location for treatment within the vasculature of a patient.

In one presently preferred embodiment, the means for dislodging the proximal end of the endoluminal device from the inner lumen of the tubular distal tip comprises an elongated pusher member coaxially disposed within the elongated flexible tubular catheter having proximal and distal ends, with the proximal end of the pusher member extending from the proximal end of the elongated flexible tubular catheter, and the distal end of the pusher member being adapted to contact and dislodge the proximal end of the endoluminal device from the tubular distal tip. The distal end of the elongated flexible tubular catheter has a frustoconical shape, and the distal end of the pusher member has a frustoconical shape.

A flexible coil is mounted to the distal end of the elongated pusher member. The flexible coil can be formed from a shape memory polymer, a nickel titanium alloy, stainless steel, platinum, or other similar suitable materials.

In an alternative arrangement not being part of the present invention, the tubular distal tip forms a fluid seal about the proximal end of the endoluminal device, and the means for dislodging comprises a syringe connectable to the proximal end of the elongated flexible tubular catheter for supplying pressurized fluid within the elongated flexible tubular catheter to expel the proximal end of the endoluminal device from the tubular distal tip.

The diameter of the distal end of the tubular distal tip may be smaller than the proximal end, allowing the proximal end of the endoluminal device to be captured within the inner lumen of the tubular distal tip.

The yieldable material forming the tubular distal tip can comprise a shape memory material, such as a shape memory polymer, a nickel titanium alloy, an elastomer such as polyurethane, nylon, polybutyl terephthalate (PBT), polymers available under the trade names PEBAX, Hytrel, Arnitel, Riteflex, or other similar suitable yieldable materials.

The endoluminal therapeutic device has a stem portion with an enlarged proximal end captured within the inner lumen of the tubular distal tip, and is typically an embolic coil, although the endoluminal therapeutic device can also be a stent, intravascular vena cava filter, or similar device to be implanted at a treatment site in the vasculature of a patient.

A method of delivering the endoluminal therapeutic device of the present invention into the vasculature of a patient, comprises the steps of providing an elongated flexible tubular catheter having a tubular distal tip mounted to the distal end of the catheter, the tubular distal tip having an inner lumen and a distal end with a surface defining a distal opening, and the tubular distal tip being formed of a yieldable material for releasably holding the proximal end of the endoluminal device within the inner lumen of the tubular distal tip; and introducing a dislodging element into the proximal end of the elongated flexible catheter to dislodge the proximal end of the endoluminal device from the tubular distal tip to expel the proximal end of the endoluminal device through the distal opening of the tubular distal tip at the desired location for treatment within the vasculature of a patient. The step of introducing a dislodging element may comprise introducing an elongated pusher member coaxially within the elongated flexible tubular catheter to contact and dislodge the proximal end of the endoluminal device from the tubular distal tip. In an alternative arrangement, when the distal tip forms a fluid seal about the proximal end of the endoluminal device, and the step of introducing a dislodging element can comprise connecting a syringe to the proximal end of the elongated flexible tubular catheter for supplying pressurized fluid within the elongated flexible tubular catheter to expel the proximal end of the endoluminal device from the tubular distal tip.

Advantageously, the endoluminal therapeutic device is an embolic coil.

Advantageously, the endoluminal therapeutic device has a stem portion with an enlarged proximal end captured within said inner lumen of said tubular distal tip.

These and other aspects and advantages of the invention will become apparent from the following detailed description and the accompanying drawings, which illustrate by way of example the features of the invention.

### BRIEF DESCRIPTION OF THE DRAWINGS

Figure 1 is a cross-sectional view of an arrangement of an endoluminal device delivery assembly;
Fig. 2 is a side elevational view of an arrangement of the pusher member of the endoluminal device delivery assembly of Fig. 1;
Fig. 3 is a side elevational view of an embodiment of the pusher member of the endoluminal device delivery assembly of the invention; and
Fig. 4 is a sectional view of a syringe for use in combination with the catheter of the endoluminal device delivery assembly in another arrangement.

### DETAILED DESCRIPTION OF THE PREFERRED EMBODIMENTS

As very small medical instruments such as microcatheters have become available, physicians are now able to reach areas within the human body which were previously inaccessible. Among the areas which can now be accessed in a minimally invasive procedure are the tiny blood vessels within the brain. Using very small diameter pushers, it is now possible to insert therapeutic devices through microcatheters to treat damaged vasculature within the brain such as intracranial aneurysms. However, because the pushers and catheters used to deliver these devices are so small, there are practical limitations associated with their use. For example, because of the size and delicacy of the devices, it is not practical to have a device with complex moving parts at the distal end of the pusher, even though it is important to be able to reliably release the device from the pusher into the vasculature. While various methods for delivery of endoluminal devices to a treatment site within the vasculature have been developed, there remains a need for a reliable method of delivering and placing such devices, particularly into smaller, previously inaccessible areas of the vasculature.

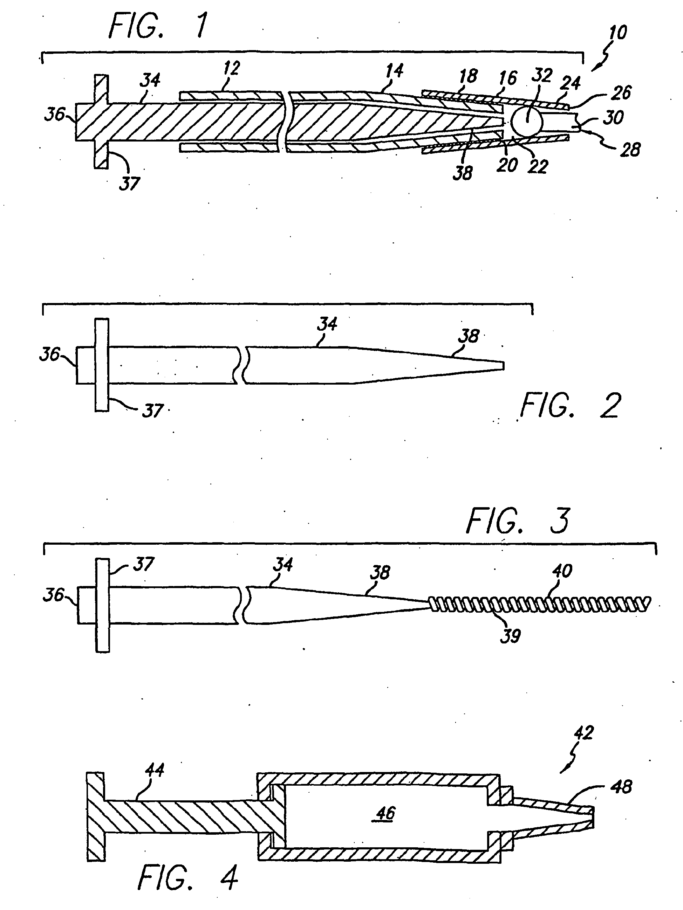

As is illustrated in the drawings, which are provided by way of example and not by way of limitation, the invention is embodied in an assembly for release and deployment of an endoluminal therapeutic device at a desired location for treatment within the vasculature of a patient. Referring to Fig.1, the endoluminal device delivery assembly 10 includes an elongated flexible tubular catheter 12 having a distal end 14. The catheter can be formed, for example, from polyethylene, polyethylene terephthalate, polyvinyl chloride, nylon and ionomers, or other similar suitable polymers, stainless steel or nickel titanium alloy hypo tubes, and the like. In a preferred arrangement, the distal end of the elongated flexible tubular catheter preferably has a frustoconical shape. The catheter advantageously includes a tubular distal tip 16 having a proximal end 18 mounted to the outer surface of the distal end of the catheter, such as by adhesive bonding, such as with acyanoacrylate adhesive 20, for example. The tubular distal tip may alternatively be heat bonded to the distal end of the catheter, or may be mounted to the distal end of the catheter by other suitable means. The tubular distal tip has an inner lumen 22, and a distal end 24 with a surface defining a distal opening 26. In one presently preferred arrangement, the diameter of the distal end of the tubular distal tip is smaller than the proximal end, allowing the proximal end of the endoluminal device to be captured within the inner lumen of the tubular distal tip. The elongated flexible tubular catheter preferably has a frustoconical distal end, and the distal tip has a corresponding frustoconical shape. In an alternative arrangement, a cylindrical tubular shape for the distal end of the catheter and the tubular distal tip may also be suitable.

The tubular distal tip is preferably formed of a yieldable material that is sufficiently rigid to retain the proximal end 28 of an endoluminal device within the inner lumen of the tubular distal tip. The yieldable material can be, for example, a shape memory polymer, an elastomer such as polyurethane, nylon, PEBAX polymer, Teloflex, polybutyl terephthalate (PBT), polymers available under the trade names PEBAX, Hytrel, Arnitel, Riteflex, heat shrink tubing such as polyethylene terephthalate (PET) or high density polyethylene (HDPE), or a shape memory metal such as nickel titanium alloy, such as that available under the trade name NITINOL

The proximal end of the endoluminal therapeutic device preferably has a stem portion 30 with an enlarged proximal end such as a ball 32, coil, block or the like, captured within the inner lumen of the tubular distal tip, and the endoluminal therapeutic device is typically an embolic coil, although the endoluminal therapeutic device can also be a stent, or a similar device to be implanted at a treatment site in the vasculature of a patient.

Means are also provided for dislodging the proximal end of the endoluminal device captured in the inner lumen of the tubular distal tip to expel the proximal end of the endoluminal device from the distal opening of the tubular distal tip at the desired location for treatment within the vasculature of a patient. As is illustrated in Figs.1 and 2, in one arrangement the means for dislodging the proximal end of the endoluminal device from the inner lumen of the tubular distal tip comprises an elongated flexible pusher member 34 such as a flexible metal wire coaxially disposed within the elongated flexible tubular catheter. The proximal end 36 of the pusher member extends from the proximal end of the elongated flexible tubular catheter, and preferably includes a stop portion 37 at the proximal end of the pusher member for limiting the movement of the pusher member through the delivery catheter, and the distal end 38 of the pusher member is adapted to contact and dislodge the proximal end of the endoluminal device from the tubular distal tip. As noted above, in one arrangement, the distal end of the elongated flexible tubular catheter is narrowed, and preferably has a frustoconical shape for improved tracking of the endoluminal device delivery assembly, and the distal end of the pusher member has a corresponding frustoconical shape, so as to extendable to the distal end of the catheter to force the proximal end of the endoluminal device from the yieldable tubular distal tip to dislodge the proximal end of the endoluminal device.

In a presently preferred embodiment illustrated in Fig. 3, the distal end 38 of the pusher member 34 has a narrowed distal cylindrical tip 39, and a flexible coil 40 is mounted to the distal tip 39 of the elongated pusher member, such as by an adhesive such as cyanoacrylate, or heat bonding, and when the coil is formed of metal, by solder, welding, or the like. The flexible coil contacts the proximal end of the endoluminal device when the pusher member is moved distally through the delivery catheter, and reduces stiffness at the distal end of the pusher member, allowing for improved tracking of the endoluminal device delivery assembly within the vasculature, and can be formed from a shape memory polymer, a nickel titanium alloy, stainless steel, or platinum, for example, or other similar suitable materials.

In another arrangement, the tubular distal tip is dimensioned so as to form a tight fluid seal about the proximal end of the endoluminal device, and the means for dislodging the endoluminal device comprises a syringe 42 having a plunger 44 for pressurizing fluid, such as saline, for example, in a fluid chamber 46 to supply pressurized fluid through a nozzle 48 that can be connected to the proximal end of the elongated flexible tubular catheter for supplying the pressurized fluid within the elongated flexible tubular catheter to expel the proximal end of the endoluminal device from the tubular distal tip.

In the method of delivering an endoluminal therapeutic device into the vasculature of a patient, the dislodging element, such as the pusher member, other similar mechanical device, or the pressurized fluid from the syringe, is introduced into the proximal end of the elongated flexible catheter to dislodge the proximal end of the endoluminal device from the tubular distal tip to expel the proximal end of the endoluminal device through the distal opening of the tubular distal tip at the desired location for treatment within the vasculature of a patient. The elongated pusher member thus can be introduced and moved coaxially distally within the elongated flexible tubular catheter to contact and dislodge the proximal end of the endoluminal device from the tubular distal tip. Although in this arrangement a fluid seal need not be formed by the tubular distal tip over the proximal stem portion of the endoluminal device, when the distal tip is dimensioned to form a fluid seal about the proximal end of the endoluminal device, and a syringe or a similar device can be connected to the proximal end of the elongated flexible tubular catheter for supplying pressurized fluid within the elongated flexible tubular catheter to force the yieldable tubular distal tip of the catheter open to expel the proximal end of the endoluminal device from the tubular distal tip, to release the endoluminal device at the desired treatment site.

It will be apparent from the foregoing that while particular forms of the invention have been illustrated and described, various modifications can be made without departing from the scope of the invention. Accordingly, it is not intended that the invention be limited, except as by the appended claims.

## Claims

1. An endoluminal device delivery assembly (10) for release and deployment of an endoluminal therapeutic device from the endoluminal device delivery assembly (10) at a desired location for treatment within the vasculature of a patient, the endoluminal therapeutic device having proximal and distal portions, the endoluminal device delivery assembly (10) comprising;
an elongated flexible tubular catheter (12) having a proximal end and a distal end (14);
an elongated pusher member (34) for dislodging the proximal end (28) of the endoluminal device from said endoluminal device delivery assembly (10) at the desired location for treatment within the vasculature of a patient, said elongated pusher member (34) being coaxially disposed within the elongated flexible tubular catheter (12), said elongated pusher member (34) having proximal (36) and distal (38) ends, said distal end (38) having a narrowed distal cylindrical tip (39), and said proximal end (36) of said pusher member (34) extending from said proximal end (12) of said elongated flexible tubular catheter (12);
a flexible coil (40) mounted to the narrowed distal cylindrical tip (39) of said pusher member (34), said endoluminal device delivery assembly (10) being **characterized by**;
a tubular distal tip (16) having a proximal end (18) mounted to the distal end (14) of said catheter, said elongated flexible tubular catheter (12) having a frustoconical distal end (14), and said tubular distal tip (16) having a frustoconical shape corresponding to the frustoconical distal end (14) of the elongated flexible tubular catheter (12), said tubular distal tip (16) having an inner lumen (22) and a distal end (24) with a surface defining a distal opening (26), and said tubular distal tip (16) being formed of a yieldable material for releasably holding the proximal end (28) of the endoluminal device within the inner lumen (22) of said tubular distal tip; and
said flexible coil (40) being adapted to contact and dislodge the proximal end of said endoluminal device from said tubular distal tip.

2. The endoluminal device delivery assembly (10) of claim 1, wherein said distal end (38) of said pusher member (34) has a frustoconical shape proximal to said narrowed distal cylindrical tip (39).

3. The endoluminal device delivery assembly (10) of either claim 1 or claim 2, wherein said flexible coil (40) is formed from a shape memory polymer.

4. The endoluminal device delivery assembly (10) of either claim 1 or claim 2, wherein said flexible coil is formed from a nickel titanium alloy.

5. The endoluminal device delivery assembly (10) of any preceding claim, wherein said yieldable material comprises a shape memory material.

6. The endoluminal device delivery assembly (10) of Claim 5, wherein said shape memory material is a shape memory polymer.

7. The endoluminal device delivery (10) of Claim 5, wherein said shape memory material is a nickel titanium alloy.

8. The endoluminal device delivery assembly (10) of any of claims 1 to 4, wherein said yieldable material is an elastomer.

9. The endoluminal device delivery assembly (10) of any of claims 1 to 4, wherein said yieldable material is selected from the group consisting of polyurethane, nylon and polybutyl terephthalate.

10. The endoluminal device delivery assembly (10) of any preceding claim, wherein said endoluminal therapeutic device is an embolic coil.

11. The endoluminal device delivery assembly of any of Claims 1 to 9, wherein said endoluminal therapeutic device has a stem portion (30) with an enlarged proximal end (30) captured within said inner lumen (22) of said tubular distal tip (16).

## Patentansprüche

1. Einbringungsanordnung für eine Endoluminalvorrichtung (10) zur Freigabe und Anbringung einer therapeutischen Endoluminalvorrichtung von der Einbringungsanordnung für eine Endoluminalvorrichtung (10) an einer gewünschten Stelle für die Behandlung in dem Gefäßsystem eines Patienten, wobei die therapeutische Endoluminalvorrichtung einen proximalen und einen distalen Teil aufweist, wobei die Einbringungsanordnung für eine Endoluminalvorrichtung (10) umfasst:
einen länglichen, flexiblen, rohrförmigen Katheter (12) mit einem proximalen Ende und einem distalen Ende (14);
ein längliches Schiebeelement (34) zum Verlagern des proximalen Endes (28) der Endoluminalvorrichtung von der Einbringungsanordnung für eine Endoluminalvorrichtung (10) an der gewünschten Behandlungsstelle im Gefäßsystem eines Patienten, wobei das längliche, flexible Schiebeelement (34) koaxial in dem länglichen, flexiblen rohrförmigen Katheter (12) angeordnet ist, wobei das längliche, flexible Schiebeelement (34) ein proximales (36) und ein distales (38) Ende umfasst, wobei das distale Ende (38) eine verengte distale zylindrische Spitze (39) aufweist und sich das proximale Ende (36) des Schiebeelements (34) von dem proximalen Ende (12) des länglichen, flexiblen, rohrförmigen Katheters (12) erstreckt,
eine flexible Spule (40), die an der verengten distalen zylindrischen Spitze (39) des Schiebeelements (34) angebracht ist, wobei die Einbringungsanordnung für eine Endoluminalvorrichtung (10) **dadurch gekennzeichnet ist, dass**:
eine rohrförmige distale Spitze (16) ein proximales Ende (18) umfasst, das an dem distalen Ende (14) des Katheters angebracht ist, wobei der längliche, flexible, rohrförmige Katheter (12) ein kegelstumpfförmiges distales Ende (14) aufweist und die rohrförmige distale Spitze (16) entsprechend dem kegelstumpfförmigen distalen Ende (14) des länglichen flexiblen rohrförmigen Katheters (12) eine kegelstumpfartige Form aufweist,
wobei die rohrförmige distale Spitze (16) ein inneres Lumen (22) und ein distales Ende (24) mit einer Fläche umfasst, die eine distale Öffnung (26) begrenzt, und wobei die rohrförmige distale Spitze (16) aus einem nachgiebigen Material gebildet ist, um das proximale Ende (28) der Endoluminalvorrichtung in dem inneren Lumen (22) der rohrförmigen distalen Spitze zu halten; und
wobei die flexible Spule (40) dazu ausgebildet ist, das proximale Ende der Endoluminalvorrichtung zu berühren und von der rohrförmigen distalen Spitze zu verlagern.

2. Einbringungsanordnung für eine Endoluminalvorrichtung (10) nach Anspruch 1, wobei das distale Ende (38) des Schiebeelements (34) nahe der verengten distalen rohrförmigen Spitze (39) eine kegelstumpfförmige Gestalt aufweist.

3. Einbringungsanordnung für eine Endoluminalvorrichtung (10) nach Anspruch 1 oder Anspruch 2, wobei die flexible Spule (40) aus einem Formgedächtnispolymer gebildet ist.

4. Einbringungsanordnung für eine Endoluminalvorrichtung (10) nach Anspruch 1 oder Anspruch 2, wobei die flexible Spule aus einer Nickel-Titan-Legierung gebildet ist.

5. Einbringungsanordnung für eine Endoluminalvorrichtung (10) nach einem der vorangehenden Ansprüche, wobei das nachgiebige Material ein Formgedächtnismaterial umfasst.

6. Einbringungsanordnung für eine Endoluminalvorrichtung (10) nach Anspruch 5, wobei das Formgedächtnismaterial ein Formgedächtnispolymer ist.

7. Einbringungsanordnung für eine Endoluminalvorrichtung (10) nach Anspruch 5, wobei das Formgedächtnismaterial eine Nickel-Titan-Legierung ist.

8. Einbringungsanordnung für eine Endoluminalvorrichtung (10) nach einem der Ansprüche 1 bis 4, wobei das nachgiebige Material ein Elastomer ist.

9. Einbringungsanordnung für eine Endoluminalvorrichtung (10) nach einem der Ansprüche 1 bis 4, wobei das nachgiebige Material gewählt ist aus der Gruppe, umfassend Polyurethan, Nylon und Polybutylterephthalat.

10. Einbringungsanordnung für eine Endoluminalvorrichtung (10) nach einem der vorangehenden Ansprüche, wobei die therapeutische Endoluminalvorrichtung eine embolische Spule ist.

11. Einbringungsanordnung für eine Endoluminalvorrichtung nach einem der Ansprüche 1 bis 9, wobei die therapeutische Endoluminalvorrichtung einen Stammabschnitt (30) mit einem vergrößerten proximalen Ende (30) umfasst, das in dem inneren Lumen (22) der rohrförmigen distalen Spitze (16) gefangen ist.

## Revendications

1. Ensemble de distribution (10) d'un dispositif endoluminal pour la libération et le déploiement d'un dispositif thérapeutique endoluminal de l'ensemble de distribution (10) du dispositif endoluminal à un emplacement souhaité pour un traitement dans le système vasculaire d'un patient, le dispositif thérapeutique endoluminal ayant des parties proximale et distale, l'ensemble de distribution (10) du dispositif endoluminal comprenant :
un cathéter (12) tubulaire souple allongé ayant une extrémité proximale et une extrémité distale (14) ;
un élément poussoir allongé (34) susceptible de déplacer l'extrémité proximale (28) du dispositif endoluminal dudit ensemble de distribution (10) du dispositif endoluminal à l'emplacement désiré pour un traitement dans le système vasculaire d'un patient, ledit élément poussoir allongé (34) étant disposé de façon coaxiale dans le cathéter (12) tubulaire souple allongé, ledit élément poussoir allongé (34) ayant des extrémités proximale (36) et distale (38), ladite extrémité distale (38) ayant une pointe cylindrique distale resserrée (39), et ladite extrémité proximale (36) dudit élément poussoir (34) s'étendant de ladite extrémité proximale (12) dudit cathéter tubulaire souple allongé (12) ;
une bobine souple (40) montée sur la pointe (39) cylindrique distale resserrée dudit élément poussoir (34), ledit ensemble de distribution (10) du dispositif endoluminal étant **caractérisé par** ;
une pointe (16) distale tubulaire ayant une extrémité proximale (18) montée sur l'extrémité distale (14) dudit cathéter, ledit cathéter (12) tubulaire souple allongé ayant une extrémité distale tronconique (14), et ladite pointe (16) distale tubulaire ayant une forme tronconique correspondant à l'extrémité distale tronconique (14) du cathéter (12) tubulaire souple allongé, ladite pointe (16) distale tubulaire ayant une lumière interne (22) et une extrémité distale (24) avec une surface définissant une ouverture distale (26), et ladite pointe (16) distale tubulaire étant formée d'un matériau déformable pour maintenir de manière libérable l'extrémité proximale (28) du dispositif endoluminal dans la lumière interne (22) de ladite pointe distale tubulaire ; et
ladite bobine souple (40) étant adaptée à rentrer en contact avec et à déplacer l'extrémité proximale dudit dispositif endoluminal de ladite pointe distale tubulaire.

2. Ensemble de distribution (10) d'un dispositif endoluminal de la revendication 1, dans lequel ladite extrémité distale (38) dudit élément poussoir (34) a une forme tronconique proximale par rapport à ladite pointe (39) cylindrique distale resserrée.

3. Ensemble de distribution (10) d'un dispositif endoluminal de l'une ou l'autre de la revendication 1 ou de la revendication 2, dans lequel ladite bobine souple (40) est formée d'un polymère à mémoire de forme.

4. Ensemble de distribution (10) d'un dispositif endoluminal de l'une ou l'autre de la revendication 1 ou de la revendication 2, dans lequel ladite bobine souple est formée d'un alliage de nickel et de titane.

5. Ensemble de distribution (10) d'un dispositif endoluminal de l'une des revendications précédentes, dans lequel ledit matériau déformable comprend un matériau à mémoire de forme.

6. Ensemble de distribution (10) d'un dispositif endoluminal de la revendication 5, dans lequel ledit matériau à mémoire de forme est un polymère à mémoire de forme.

7. Ensemble de distribution (10) d'un dispositif endoluminal de la revendication 5, dans lequel ledit matériau à mémoire de forme est un alliage de nickel et de titane.

8. Ensemble de distribution (10) d'un dispositif endoluminal de l'une des revendications 1 à 4, dans lequel ledit matériau déformable est un élastomère.

9. Ensemble de distribution (10) d'un dispositif endoluminal de l'une des revendications 1 à 4, dans lequel ledit matériau déformable est sélectionné dans le groupe constitué du polyuréthane, du nylon et du polytéréphtalate de butylène.

10. Ensemble de distribution (10) d'un dispositif endoluminal de l'une des revendications précédentes, dans lequel ledit dispositif thérapeutique endoluminal est une bobine de Löhlein.

11. Ensemble de distribution d'un dispositif endoluminal de l'une des revendications 1 à 9, dans lequel ledit dispositif thérapeutique endoluminal a une partie de tige (30) avec une extrémité proximale agrandie (30) capturée dans ladite lumière interne (22) de ladite pointe distale tubulaire (16).
